# EUROPÄISCHE PATENTSCHRIFT

(11) **EP 1 960 489 B1**
(45) Veröffentlichungstag und Bekanntmachung des Hinweises auf die Patenterteilung: **22.04.2009**
(21) Anmeldenummer: 06806862.6
(22) Anmeldetag: 28.09.2006
(51) Int. Cl.: C09J 133/00, A61L 15/48

(54) **ACRYLATKLEBEMASSE MIT WASSERFESTEN KLEBEIGENSCHAFTEN**
ACRYLATE ADHESIVE WITH WATER-RESISTANT BONDING PROPERTIES
MATIERE ADHESIVE A BASE D ACRYLATE DONT LES PROPRIETES ADHESIVES RESISTENT A L EAU

(30) Priorität: 08.12.2005 DE 102005059058
(43) Veröffentlichungstag der Anmeldung: 27.08.2008
(73) Patentinhaber: Beiersdorf AG, 20253 Hamburg (DE)
(72) Erfinder: MEYER-INGOLD, Wolfgang, 22457 Hamburg (DE)
(86) Internationale Anmeldenummer: PCT/EP2006/066835
(87) Internationale Veröffentlichungsnummer: WO 2007/065742

(56) Entgegenhaltungen:
- LEE B P ET AL: "Synthesis of 3,4-dihydroxyphenylalanine (DOPA) containing monomers and their copolymerization with PEG-diacrylate to form hydrogels" JOURNAL OF BIOMATERIALS SCIENCE, POLYMER EDITION VSP NETHERLANDS, Bd. 15, Nr. 4, 2004, Seiten 449-464, XP009075020 ISSN: 0920-5063 in der Anmeldung erwähnt
- DALSIN ET AL: "Bioinspired antifouling polymers" MATERIALS TODAY, ELSEVIER SCIENCE, KIDLINGTON, GB, Bd. 8, Nr. 9, September 2005 (2005-09), Seiten 38-46, XP005024450 ISSN: 1369-7021

## Beschreibung

Die Erfindung umfasst eine Acrylatklebemasse und diese enthaltende Pflaster bzw. Hautauflagen, deren Klebkraft auch unter feuchten Bedingungen keine Verluste erleidet.

Klebemassen finden bei der Herstellung von Wundversorgungsprodukten ein breites. Anwendungsspektrum, wenn sie aufgrund eines hohen Polymerisationsgrades nur minimale Mengen an Restmonomeren enthalten und dadurch als besonders hautverträglich gelten können.

Eine Reihe von Druckschriften offenbaren dazu verschiedenste Acrylatmassen und Kautschukmassen, wie beispielsweise, EP 352901, EP 238863, JP 1060677, US 4269747, US 5876855, JP10152662, DE 42 22 334, DE 196 31 422, DE 102 13 759, WO 9625469 oder WO9723577. Die Herstellung von Polymermatrices, insbesondere Gelmatrices, aus-Polyacrylaten ist seit vielen Jahren bekannt und wird z.B. in EP 0 507 160, JP 11-228340 und JP 04178323 beschrieben.

Hauptaufgabe der verwendeten Klebemassen ist dabei eine sichere Verklebung des Wundversorgungsproduktes mit der Haut sowie am Ende der Anwendungszeit eine leichte und insbesondere schmerzlose Entfernung von der Haut.

Diese auch bei zahlreichen Verbraucherbefragungen- immer- wieder erfahrene wichtigste Eigenschaft eines Pflasters ist allerdings nicht in allen Fällen erreichbar. Transpiration oder Schwitzen führen in Abhängigkeit von der Flüssigkeitsaufnahme-Kapazität der Wundauflage und/oder der Klebemasse sowie von der Wasserdampfdurchlässigkeit des Pflasters zu mehr oder weniger häufigen unerwünschten Ablösungen von Pflastern.

Im Stand der Technik hat es nicht an Versuchen gefehlt diesen Nachteilen.Herr zu werden und eine verbesserte, weniger feuchtigkeitsempfindliche Klebemasse zu entwicklen, wie z.B. durch den zusätzlichen Einsatz von Superabsorbern oder durch Hydrophilisierung.
Für Acrylat-Klebemassen ist der Einsatz von Hydroxyethyl-acrylat oder -methacrylat zur Hydrophilisierung beispielsweise in US 5302629 beschrieben. Klebemassen auf Basis von synthetischem Kautschuk können-durch Zugabe eines sog. Surfactantshydrophiiisiert werden, wie im US 6860961 beschrieben.
Ein weiteres Problem bei der Vernetzung von Polyacrylsäure zu einer selbstklebenden Matrix bzw. Gel ist, dass eine einmal hergestellte-Matrix-mit definierten-physikalischen Eigenschaften, Viskosität; Klebrigkeit etc. in einem spätere Herstellungsprozess die gleichen definierten Eigenschaften aufweisen muss. Diese Reproduzierbarkeit ist mit den derzeit bekannten Vernetzungstechnologien aufwändig oder gar nicht zu verwirklichen.

Bekannt ist aus einem völlig anderen technischem Gebiet, dass z.B. Seepocken und Pfahlmuscheln eine perfekte wasserfeste Klebung erzielen können. Dabei spielt 3,4-Dihydroxyphenylalanin (DOPA) eine wichtige Rolle, nachdem repetitive Sequenze von DOPA haltigen Dekapeptiden in dem Protein identifiziert worden sind, das sich für die Verklebung verantwortlich zeigt, wie in der Literatur ausgeführt wird (J. H Waite, J. Biol. Chem. 258, 2911-2915 [1983]). Der Mechanismus der Anhaftung und der dann erfolgenden festen Verklebung dieser Organismen über sogenannte MAPs ("Mussel Adhesive Proteins") ist zudem aufgeklärt worden (M. Yu et al., J. Am. Chem. Soc. 121, 5825-5826 [1999]).

Nach der Identifizierung des sogenannten Muschelklebers ist es gemäß der US 20030087338 gelungen, dieses Prinzip in synthetische Polymere einzubauen. In der US 20030087338 basiert dieses Prinzip auf dem oben beschriebenen, chemisch synthetisieren L-3,4-Dihydroxyphenylalanin enthaltendem Dekapeptid, das eine Sequenz aus den MAPs der Muschel Mytilis edulis darstellt. Dieses Dekapeptid kann an verschiedene Polymere konjugiert werden, um eine biomimetische Klebemasse zu erzeugen. Die den MAPs analoge Quervernetzung von DOPA-Gruppen zur biomimetischen Klebemasse kann dabei enzymatisch oder oxidativ erfolgen. Die oxidative Quervernetzung führt dabei allerdings zu Klebkraftverlusten. Deshalb beschreibt die US 20030087338 auch oxidationsunabhängige Aggregations- und/oder Gelierungs-Phänomene zur Herstellung entsprechender Polymere oder Copolymere. Als mögliche Polymere bzw. Copolymere werden Polyethylenglycole oder Polyalkylenoxide dargestellt. Eine Kombination mit acrylathaltigen Klebemassen wird nicht offenbart. Die beschriebene, zwingend zur Herstellung der biometischen Klebmasse notwendigen Dekapeptid-Synthese erweist sich zudem als umständlich und zeitraubend.

Aufgabe der vorliegenden Erfindung ist es Klebemassen, insbesondere Acrylatklebemassen bereit zu stellen, die eine gegenüber dem Stand der Technik verbesserte Klebkraft aufweisen und weniger feuchtigkeitsempfindlich sind. Insbesondere ist es die Aufgabe der vorliegenden Erfindung eine Klebemasse bereit zu stellen, die mit dem Prinzip der wasserfesten Klebung von Seepocken und Pfahlmuscheln kombiniert werden kann ohne das die widrigen Umstände und Nachteile der Dekapeptid-Synthese zu berücksichtigen sind.

Gelöst werden diese Aufgaben durch eine Acrylatklebemasse bzw. Pflaster und Hautauflagen diese enthaltend entsprechend den Hauptansprüchen. In den Unteransprüchen sind bevorzugte Ausführungsformen der Klebemasse bzw. der Pflaster offenbart. Die Erfindung umfasst darüber hinaus auch das Verfahren zur. Herstellung derartiger Klebemassen und Pflaster sowie- deren Verwendung.

Es war überraschend und für den Fachmann außerordentlich erstaunlich, dass eine Acrylatklebemasse umfassend N-Methacryloyl-3,4-dihydroxyphenylalanin und/oder N-Acrylol-3,4-dihydroxyphenylalanin die gestellten Aufgaben löst.

B. P. Lee et al. hat in seiner Veröffentlichung - J. Biomater. Sci. Polymer Edn. 15, 449-464 [2004] ein DOPA-haltiges Monomer, N-Methacryloyl-3,4-dihydroxyphenylalanin, erstmalig beschrieben.
Nach eingehender Untersuchung ist es nun erfindungsgemäß gelungen mit Hilfe dieses Monomers auf den umständlichen Weg der Dekapeptid-Synthese, wie sie im Stand der Technik beschrieben ist, zu verzichten.

Es hat sich überraschenderweise gezeigt; dass dieses Monomer, N-Methacryloyl-3,4-dihydroxyphenylalanin ebenso wie das analog herstellbare N-Acrylol-3,4-dihydroxyphenylalanin, als zusätzlicher Ausgangsstoff direkt in die Polymerisierungsansätze für Acrylatklebemassen eingesetzt werden kann ohne das sie ihre positiven Eigenschaften einer wasserfesten Klebung verlieren. Dies wird ermöglicht, da die anschließende Polymerisation der Monomere und der Acrylate nicht über das DOPA selbst, sondern über den Acryloyl- bzw. Methacryloyl-Teil des jeweiligen Monomers erfolgt, und, das ist der Kern der Erfindung, somit aufwendige enzymatische, oxidative oder sonstige Mechanismen zur Bildung des finalen klebenden Polymers nicht erforderlich sind.

Auf diese Weise können N-Acrytol-3,4-dihydroxyphenylalanin und/oder N-Methacryloyl-3,4-dihydroxyphenylalanin in gängigen Abmischungen von Acrylsäure und Acrylsäureestern eingebaut werden und führen dann zu wasserfesten, MAP-analogen Verklebungen der Klebemassen.
Gegenüber den bekannten MAP-Verklebungen des Standes der Technik ist es aber erstmals gelungen Acrylatklebemasse in dieser Form wasserfeste Klebeeigenschaften zu vermitteln und zudem auf komplizierte, zeitraubende und die Klebeeigenschaften verschlechternde Verfahrensschritte, wie der Dekapeptid-Synthese, bei der Herstellung zu verzichten.

Vorteilhaft ist es Gehalte an N-Acrylol-3,4-dihydroxyphenylalanin und/oder N-Methacryloyl-3,4-dihydroxyphenylalanin zwischen 5 Gew.% und 20 Gew.%, bezogen auf die Gesamtklebemasse, einzusetzen.

Die Herstellung von N-Acrylol-3,4-dihydroxyphenylalanin und N-Methacryloyl-3,4-dihydroxyphenylalanin erfolgt entsprechend den Anweisungen aus J. Biomater. Sci. Polymer Edn. 15, 449-464 [2004].

Klebemassen auf Acrylatbasis haben in der Regel zwar eine gute Durchlässigkeit für Wasserdampf, ihr Klebeverhalten ist allerdings eher moderat.

Die Verwendung von hautfreundlichen Klebemassen, wie Acrylatklebemassen, ist aufgrund der geringen Scherstabilität und Anfaßklebrigkeit nicht immer erwägenswert. Eine Verbesserung durch eine Nachbehandlung, insbesondere Vernetzung, ist möglich, jedoch bleibt das Ergebnis insgesamt unbefriedigend. Weiterhin reicht die Klebkraft auf der Trägerrückseite solcher Systeme bei zirkulär angelegten Verbänden mit mehreren Lagen nicht für einen stabilen funktionalen Verband aus. Die propriorezeptive Wirkung ist auch gegenüber den Systemen mit einer Zink-Kautschuk-Klebemasse geringer.
Andere bekannte Klebesysteme auf Basis von herkömmlichen Blockcopolymeren sind zum einen durch hohen Stabilisatorzusatz nicht hautfreundlich beziehungsweise zeigen durch diehohe Kohäsivität bislang nur eine Eignung für technische Anwendungsfälle, zum anderen sind sie nicht stärk hautklebend und hauthaftend einzustellen.
Die genannten Klebemassen, Acrylat- und Methacrylatklebemassen, zählen zu den druckempfindliche Selbstklebemassen, wobei die Massen für die Verarbeitung in einer Trägermatrix vorliegen können. Als Trägermatrix werden gängige organische oder anorganische Lösemittel oder Dispergiermittel verstanden.

Als bevorzugte Matrix kann eine Polymermatrix aus hydrophilen Polymeren, wie z.B. Acrylate, Polyvinylpyrrolidon/Polyacrylsäure oder Polyacrylsäure/Polyvinylalkohol zum Einsatz kommen.

Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eigentlich eine eingetragene Marke der B. F. Goodrich Company) gewählt werden. Insbesondere zeichnen sich das oder die erfindungsgemäß vorteilhaften Acrylat-Alkylacrylat-Copolymere durch die folgende Struktur aus:

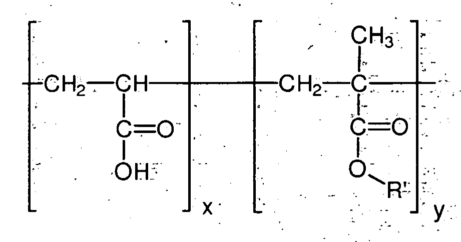

Darin stellt R' einen langkettigen Alkylrest und x und y Zahlen dar, welche den jeweiligen stöchiometrischen Anteil der jeweiligen Comonomere symbolisieren.

Erfindungsgemäß besonders bevorzugt sind Acrylat-Copolymere und/oder Acrylat-Alkylacrylat-Copolymere, welche unter den Handelbezeichnungen Carbopol® 1382, Carbopol®, 981 und Carbopol® 5984 von der B. F.Goodrich Company erhältlich sind, bevorzugt sind Polyacrylate aus der Gruppe der Carbopole der Typen 980, 981, 1382, 2984, 5984 sowie besonders bevorzugt Carbomer 2001.

Diesen Acrylat- oder Methacrylatpolymeren bzw. copolymeren werden die N-Acrylol-3,4-dihydroxyphenylalanin und/oder N-Methacryloyl-3,4-dihydroxyphenylalanin Monomere bei der Klebemassenherstellung zugesetzt.

Erhalten werden dadurch Klebemassen, die einerseits die Vorteile der Acrylatklebemasen aufweisen und andererseits auch unter Feuchtigkeitseinfluss keinerlei Klebkraftverlust erfahren. Die Kombination aus N-Acrylol-3,4-dihydroxyphenylalanin und/oder N-Methacryloyl-3,4-dihydroxyphenylalanin und Acrylat- oder Methacrylatpolymeren ermöglicht somit die Herstellung biomimetischer Klebemassen.
Bevorzugt wird bei der Herstellung auf die, wie beschrieben, nachteilige Dekapeptid-Synthese, d.h. der den MAPs, analoge Quervernetzung von DOPA-Gruppen zur biomimetischen Klebemasse auf enzymatischem oder oxidativem Weg, verzichtet.

Die biomimetischen Acrylatklebemasse als Klebemasse in Hautauflagen oder Pflaster ermöglicht die Bereitstellung von Hautauflagen oder Pflaster, die auch unter Wasser oder feuchten Bedingungen ausreichend feste Verklebung, insbsondere auf der menschlichen Haut, gewährleisten. Somit sind die Probleme bei der Pflasterhaftung auf menschlicher Haut, die beim Schwitzen, beim Waschen, bei Regen oder sonstigen feuchten Einflüssen gemeinhin auftreten, gelöst.

Pflaster umfassen neben der Klebemassen mitunter ein Trägermaterial auf dem die Klebemasse aufgetragen ist.
Als Trägermaterialien eignen sich alle starren, und elastischen Flächengebilde aus synthetischen und natürlichen Rohstoffen. Bevorzugt sind Trägermaterialien die nach Applikation der Klebemasse so eingesetzt werden können, daß sie Eigenschaften eines funktionsgerechten Verbandes, erfüllen. Beispielhaft sind Textilien wie Gewebe, Gewirke, Gelege, Vliese, Laminate, Netze, Folien, Schäume und Papiere aufgeführt. Weiter können diese Materialien vor- bzw. nachbehandelt werden. Gängige Vorbehandlungen sind Corona und Hydrophobieren; geläufige Nachbehandlungen sind Kalandern, Tempern, Kaschieren, Stanzen und Eindecken.
Die Trägermaterialien bestehen bevorzugt aus einer luft- und wasserdampfdurchlässigen aber wasserundurchlässigen Polymerschicht mit einer Dicke von ca. 10 bis 100 µm. Die u.U. flexible Trägerfolie besteht vorzugsweise aus Polymeren von Polyurethan, PE, PP, Polyamid, Polyester oder Polyether-ester oder bekannten Trägermaterialien wie Gewebe, Vliese, Schäume, Kunststoffe etc.
Die erfindungsgemäße Klebematrix kann auf dieser Trägerschicht oder -folie aufgebracht sein, wie sie aus dem Stand der Technik bekannt ist. Dabei wird die Matrix einseitig mit dem Trägermaterial abgedeckt und als Verbundfolie appliziert. Je nach verwendetem Trägermaterial können dadurch die Wasserdampfdurchlässigkeit, die Festigkeit der Wundabdeckung, die Polsterung gegen Druck und andere physikalische Eigenschaften der Wundabdeckung gesteuert werden.

Das erfindungsgemäße Verbandsmaterial ist dann entsprechend bekannten Wundverbände aufgebaut. Sie bestehen im allgemeinen aus einem Trägermaterial, das auf einer Seite mit einer selbstklebenden Schicht, der erfindungsgemäßen Klebemasse, versehen ist. Auf diese selbstklebende Beschichtung ist dann eine Wundauflage aufgebracht. Um eine einfache Handhabung zu gewährleisten, wird die selbstklebende Beschichtung darüber hinaus mit einer schützenden Schicht eingedeckt, zum Beispiel einem Siegelpapier.
Schließlich kann das selbstklebend ausgerüstete Trägermaterial nach der Applikation zusätzlich eingedeckt oder mit einer Wundauflage; Polsterung versehen werden.
Neben der Anwendung als Pflaster kann auch ein z.B. weniger klebendes Patch oder Pad mit der erfindungsemäßen Klebemasse ausgestattet sein, wobei die Klebkraft nach dem jeweiligen Anwendungszweck spezifisch eingestellt werden kann.
Die Begriffe Pflaster, kosmetische/dermatologische Matrices und kosmetische/dermatologische Pads werden vorliegend ebenso synonym benutzt, wie ebenso unter den erfindungsgemäßen Hautauflage alle kosmetisch anwendbaren Auflagen wie patch, pad, tapes, Tücher, Binden, Pflaster, dressings, Cataplasm, Bandagen und/oder Masken verstanden werden.

Es ist durch die erfindungsgemäße Klebemasse erstmals gelungen, eine Verklebung von Substraten unter feuchten Bedingungen, insbesondere eine biokompatible und wasserfeste-Verklebungen auf der menschlichen Haut zu gewährleisten.

## Patentansprüche

1. Acrylat- und/oder Methacrylatklebemasse umfassend N-Methacryloyl-3,4-dihydroxyphenylalanin und/oder N-Acrylol-3,4-dihydroxyphenylalanin.

2. Klebemasse nach Anspruch 1 umfassend zwischen 5 Gew.% und 20 Gew.%, bezogen auf die Gesamtklebemasse, N-Methacryloyl-3,4-dihydroxyphenylalanin und/oder N-Acrylol-3,4-dihydroxyphenylalanin.

3. Verfahren zur Herstellung von biokompatibler Acrylatklebemasse indem N-Methacryloyl-3,4-dihydroxyphenylalanin und/oder N-Acrylol-3,4-dihydroxyphenylalanin als Monomere in die Acrylat-und/oder Methacrylatausgangsmischung zugegeben werden.

4. Kombination aus N-Acrylol-3,4-dihydroxyphenylalanin und/oder N-Methacryloyl-3,4-dihydroxyphenylalanin und Acrylat- und/oder Methacrylatpolymeren zur Herstellung biomimetischer Klebemassen.

5. Selbstklebende Hautauflage oder Pflaster umfassend eine Klebemasse nach einem der vorstehenden Ansprüche.

6. Verwendung einer Klebemasse nach einem- der Ansprüche 1 bis 4 zur- Verklebung von-Substraten unter feuchten Bedingungen.

7. Verwendung einer Klebemasse nach Anspruch 1- bis 4 zur Verklebung von patch, pad, tapes, Tücher, Binden, Pflaster, dressings, Cataplasm, Bandagen und/oder Masken auf der menschlichen Haut.

## Claims

1. Acrylate and/or methacrylate adhesive comprising N-methacryloyl-3,4-dihydroxyphenylalanine and/or N-acryloyl-3,4-dihydroxyphenylalanine.

2. Adhesive according to Claim 1, comprising between 5% and 20% by weight, based on the total adhesive, of N-methacryloyl-3,4-dihydroxyphenylalanine and/or N-acryloyl-3,4-dihydroxyphenylalanine.

3. Method of producing biocompatible acrylate adhesive by adding N-methacryloyl-3,4-dihydroxy-phenylalanine and/or N-acryloyl-3,4-dihydroxy-phenylalanine as monomers to the initial acrylate and/or methacrylate mixture.

4. Combination of N-acryloyl-3,4-dihydroxy-phenylalanine and/or N-methacryloyl-3,4-dihydroxy-phenylalanine and acrylate and/or methacrylate polymers for producing biomimetic adhesives.

5. Self-adhesive skin contact material or plaster comprising an adhesive according to any of the preceding claims.

6. Use of an adhesive according to any of Claims 1 to 4 for bonding substrates under moist conditions.

7. Use of an adhesive according to Claim 1 to 4 for bonding patches, pads, tapes, wipes, towels, plasters, dressings, cataplasms, bandages and/or masks on the human skin.

## Revendications

1. Masse adhésive à base d'acrylate et/ou de méthacrylate comprenant de la N-méthacryloyl-3,4-dihydroxyphénylalanine et/ou de la N-acryloyl-3,4-dihydroxyphénylalanine.

2. Masse adhésive selon la revendication 1 comprenant entre 5% en poids et 20% en poids, par rapport à la masse adhésive totale, de N-méthacryloyl-3,4-dihydroxyphénylalanine et/ou de N-acryloyl-3,4-dihydroxyphénylalanine.

3. Procédé pour la préparation d'une masse adhésive biocompatible à base d'acrylate en ce qu'on ajoute de la N-méthacryloyl-3,4-dihydroxyphénylalanine et/ou de la N-acryloyl-3,4-dihydroxyphénylalanine comme monomère dans le mélange de départ à base d'acrylate et/ou de méthacrylate.

4. Combinaison de N-acryloyl-3,4-dihydroxyphénylalanine et/ou de N-méthacryloyl-3,4-dihydroxyphénylalanine et de polymères d'acrylate et/ou de méthacrylate pour la préparation de masses adhésives biomimétiques.

5. Emplâtre ou pansement autoadhésif comprenant une masse adhésive selon l'une quelconque des revendications précédentes.

6. Utilisation d'une masse adhésive selon l'une quelconque des revendications 1 à 4 pour le collage de substrats dans des conditions humides.

7. Utilisation d'une masse adhésive selon la revendication 1 à 4 pour le collage de patches, de compresses, de tapes, de draps, de serviettes, de pansements, de sparadraps, de cataplasmes, de bandages et/ou de masques sur la peau humaine.
